(19) Europäisches Patentamt
European Patent Office
Office européen des brevets

(11) EP 1 473 568 A2

(12) EUROPEAN PATENT APPLICATION

(43) Date of publication:
03.11.2004 Bulletin 2004/45

(51) Int Cl.7: G01N 33/86, G01N 33/49

(21) Application number: 04010359.0

(22) Date of filing: 30.04.2004

(84) Designated Contracting States:
AT BE BG CH CY CZ DE DK EE ES FI FR GB GR
HU IE IT LI LU MC NL PL PT RO SE SI SK TR
Designated Extension States:
AL HR LT LV MK

(30) Priority: 02.05.2003 US 428708
12.09.2003 US 662043

(71) Applicant: WADA, Inc.
Santa Barbara, CA 93108 (US)

(72) Inventors:
• Carroll, Wallace E.
  Santa Barbara California 93108 (US)
• Jackson, David R.
  Rio Rancho New Mexico 87124 (US)

(74) Representative: Bressel, Burkhard, Dr.
Effert, Bressel und Kollegen,
Patentanwälte,
Radickestrasse 48
12489 Berlin (Adlershof) (DE)

(54) Method and apparatus for determining anticoagulant therapy factors

(57) A method and apparatus are disclosed for determining a new anticoagulant therapy factor (nATF) for monitoring oral anticoagulant therapy to help prevent excessive bleeding or deleterious blood clots that might otherwise occur before, during or after surgery. The new anticoagulant therapy factor (nATF) is based upon a determination of the new fibrinogen transformation rate (nFTR) which, in turn, is dependent on a maximum acceleration point (MAP) for fibrinogen (FBG) conversion.

The nATF quantity is also based upon the time to maximum acceleration from the time of reagent injection (TX) into a plasma sample, but does not require the difficulty of obtaining prior art International Normalized Ratio (INR) and International Sensitivity Index (ISI) parameters. The International Normalized Ratio (INR) was created to relate all species' clotting material to human clotting material, and nATF can replace INR in anticoagulant therapy management.

FIG. 2

**Description**

This invention relates to analyzing blood for carrying out coagulation studies and other chemistry procedures, including monitoring oral anticoagulant therapy to take into account the platelet count in determining prothrombin times (PT), and a new Anticoagulant Therapy Factor (nATF)

Testing of blood and other body fluids is commonly donc in hospitals, labs, clinics and other medical Facilities For example, to prevent excessive bleeding or deleterious blood clots, a patient may receive oral anticoagulant therapy before, during and after surgery To assure that the oral anticoagulant therapy is properly administered, strict monitoring is accomplished and is more fully described in various medical technical literature, such as the articles entitled "PTs, PR, ISIs and iNRs: A Primer on Prothrombin Time Reporting Parts I and II" respectively published November, 1993 and December, 1993 issues of Clinical Hemostasis Review, and herein incorporated by reference.

These technical articles disclose anticoagulant therapy monitoring that takes into account three parameters which are. International Normalized Ratio (INR), International Sensitivity Index (ISI) and prothrombin time (PT), reported in seconds The prothrombin time (PT) indicates the level of prothrombin and blood factors V, VII, and X in a plasma sample and is a measure of thc coagulation response of a patient. The INR and ISI parameters are needed so as to take into account various differences in instrumentation, methodologies and in thromboplastins (Tps) sensitivities used in anticoagulant therapy In general, thromboplastins (Tps) used in North America are derived from rabbit brain, those previously used in Great Britain from human brain, and those used in Europe from either rabbit brain or bovine brain The INR and ISI parameters take into account all of these various factors, such as the differences in thromboplastins (Tps), to provide a standardized system for monitoring oral anticoagulant therapy to reduce serious problems related to prior, during and after surgery, such as excessive bleeding or the formation of blood clots.

As reported in Part I (Calibration of Thromboplastin Reagents and Principles of Prothrombin Time Report) of the above technical article of the Clinical Hemostasis Review, the determination of the INR and ISI parameters are quite involved, and as reported in Part II (Limitation of INR Reporting) of the above technical article of the Clinical Hemostasis Review, the error yielded by the INR and ISI parameters is quite high, such as about 13%. The complexity of the interrelationship between the International Normalized Ratio (INR), the International Sensitivity Index (ISI) and the patient's prothrombin time (PT) may be given by the below expression (1), wherein the quantity

$$PR= \left[ \frac{Patient's\ PT}{Mean\ of\ PT\ Normal\ Range} \right]$$

is commonly referred to as prothromhin ratio (PR):

$$INR = \left[ \frac{Patient's\ PT}{Mean\ of\ PT\ Normal\ Range} \right]^{ISI} \quad (1)$$

The possible error involved with the use of International Normalized Ratio (INR) is also discussed in the technical article entitled "Reliability and Clinical Impact of the Normalization of the Prothrombin Times in Oral Anticoagulant Control" of E A Loeliger et al, published in Thrombosis and Hemostasis 1985; 53: 148-154, and herein incorporated by reference As can be seen in expression (1), ISI is an exponent of INR which leads to the possible error involved in the use of INR to be about 13.5% or possibly even more. A procedure related to the calibration of the ISI is described in a technical article entitled "Failure of the International Normalized Ratio to Generate Consistent Results within a Local Medical Community" of V.L. Ng et al., published in Am J. Clin Pathol 1993; 99; 689-694, and herein incorporated by reference.

The unwanted INR deviations are further discussed in the technical article entitled "Minimum Lyophilized Plasma Requirement for ISI Calibration" of L. Roller et al published in Am.J.Clin.Pathol. February 1998, Vol 109, No.. 2, 196-204, and herein incorporated by reference As discussed in this article, the INR deviations became prominent when the number of abnormal samples being tested therein was reduced to fewer than 20 which leads to keeping the population of the samples to at least 20 The paper ofL. Poller ct al. also discusses the usage of 20 high lyophilized INR plasmas and 7 normal lyophilized plasmas to calibrate the INR. Further, in this article, a deviation of +/- 10% from means was discussed as being an acceptable limit of INR deviation. Further still, this article discusses the evaluation techniques of taking into account the prothrombin ratio (PR) and the mean normal prothrombin time (MNPT), i.e.., the geometric mean of normal plasma samples.

The discrepancies related to the use of the INR are further studied and described in the technical article of V L.. NG et al- entitled, "Highly Sensitive Thromboplastins Do Not Improve INR Precision," published in Am.J.Clin.Pathol., 1998; 109, No. 3, 338-346 and herein incorporated by reference.. In this article, the clinical significance of INR discordance is examined with the results being tabulated in Table 4 therein and which arc analyzed to conclude that the level of discordance for paired values of individual specimens tested with different thromboplastins disadvantageously range from 17% to 29%.

U.S Patent 5.981,285 issued on November 9,1999 to Wallace E Carroll et al , which discloses a "Method and Apparatus for Determining Anticoagulant Therapy Factors" provides an accurate method for taking into account varying prothrombin times (PT) caused by different sensitivities of various thromboplastin formed from rabbit brain, bovine brain or other sources used for anticoagulant therapy This method does not suffer from the relatively high (13%) error sometimes occurring because of the use of the OR and ISI parameters with the exponents used in their determination.

This invention relates to the inventions disclosed in U.S Patent Nos 3,905,769 ('769) of Sep 16,1975; 5,197,017 ('017) dated Mar 23, 1993; and 5,502,651 ('651) dated Mar. 26, 1996, all issued to Wallace E- Carroll and R David Jackson, and all of which are incorporated herein by reference The present invention provides an apparatus and method for monitoring anticoagulant therapy The problem is solved by the method according to claim 1 and the apparatus according to claim 10. Preferred embodiments of the invention are quoted in the sub-claims.

The method and apparatus according to the present invention are useful for processing coagulation studies, and other chemistry procedures involving blood and blood components The apparatus and method, in accordance with a preferred embodiment of the present Invention, arc used to determine anticoagulant therapy factors which are designated herein, in particular, to determine new Anticoagulant Therapy Factor (nATF) which preferably may replace International Normalized Ratio (INR) in anticoagulation therapy management. Previously, anticoagulation therapy involved the use of International Normalized Ratios (INR's). The International Normalized Ratio (INR) was utilized in order to arrive at an anticoagulant therapy factor (ATF), which was dependent on the prothrombin time (PT), the prothrombin ratio (PR), a fibrinogen transformation rate (FTR), and a maximum acceleration point (MAP) having an associated time to maximum acceleration (TMA).

A method and apparatus are disclosed for determining a new anticoagulant therapy factor (nATF) for monitoring oral anticoagulant therapy to help prevent excessive bleeding or deleterious blood clots that might otherwise occur before, during or after surgery The new anticoagulant therapy factor (nATF) is based upon a determination of the fibrinogen transformation rate (FTR) which, in turn, is dependent on a maximum acceleration point (MAP) for fibrinogen (FBG) conversion The nATF quantity is also based upon the time to maximum acceleration from the time of reagent injection (TX) into a plasma sample, but does not require the difficulty of obtaining prior art International Normalized Ratio (INR) and International Sensitivity Index (ISI) parameters The International Normalized Ratio (INR) was created to relate all species' clotting material to human clotting material, and nATF can replace TNR in anticoagulant therapy management.

In accordance with the present invention, there is provided an apparatus and method for carrying out coagulation studies and other chemical procedures and analyses.

Fig. 1 is a diagram of potentiophotometric apparatus constructed in accordance with the present invention for determining blood chemistry analyses such as coagulation studies, including determination of the new anticoagulant therapy factor (nATF), where the output of the analog/digital (A/D) converter is applied to a computer

Fig. 2 is a plot of the various phases of the fibrinogen concentration occurring in a typical plasma clotting process

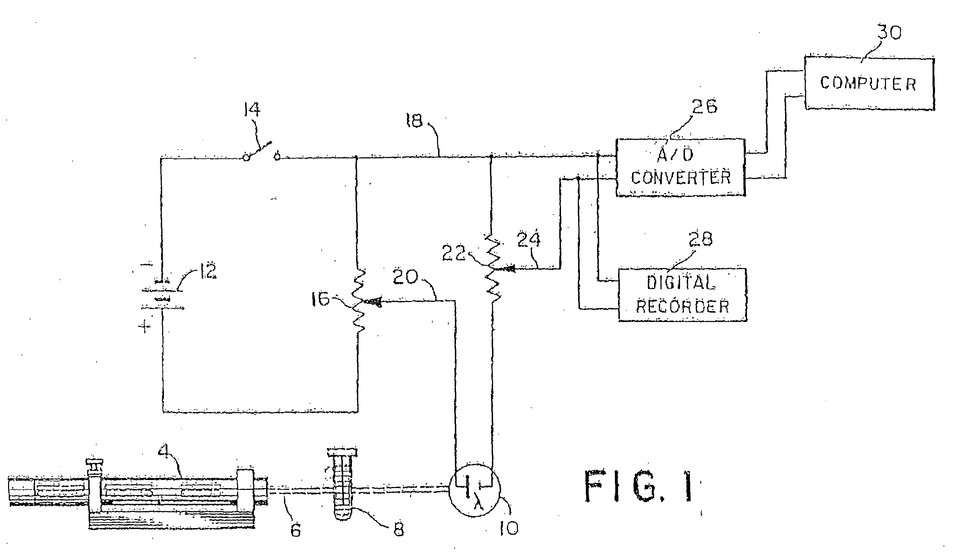

Referring to the drawings, wherein the same reference numbers indicate the same elements throughout, there is shown in Fig. 1 a light source 4 which may be a low power gas laser, or other light producing device, producing a beam of light 6 which passes through a sample test tube, such as the container 8, and is received by detection means which is preferably a silicon or selenium generating photocell 10 (photovoltaic cell). Battery 12 acts as a constant voltage DC source Its negative terminal is connected through switch 14 to one end of variable resistor 16 and its positive terminal is connected directly to the opposite end of variable resistor 16. The combination of battery 12 and variable resistor 16 provides a variable DC voltage source, the variable voltage being derivable between line 18 at the upper terminal of resistor 16 and wiper 20- This variable DC voltage source is connected in series with detection means photocell 10, the positive output of detection means photocell 10 being connected to the wiper 20 of variable resistor 16 so that the voltage produced by the variable voltage DC source opposes the voltage produced by the detection means photocell 10. The negative output of detection means photocell 10 is connected through variable resistor 22 to line 18 Thus, the voltage across variable resistor 22 is the difference between the voltage produced by the variable voltage DC source and the voltage produced by the photovoltaic cell 10 The output of the electrical network is taken between line 18 and wiper 24 of variable resistor 22. Thus, variable resistor 22 acts as a multiplier, multiplying the voltage produced as a result of the aforesaid subtraction by a selective variable depending on the setting of variable resistor-22 The potentiophotometer just described embodies the electrical-analog solution to Beer's Law and its output is expressed directly in the concentration of the substance being measured.

Wiper 24 is illustrated placed at a position to give a suitable output and is not varied during the running of the test. The output between line 18 and wiper 24 is delivered to an A/D converter 26 and digital recorder 28. As is known, the A/D converter 26 and the digital recorder 28 may be combined into one piece of equipment and may, for example, be a device sold commercially by National instrument of Austin, Texas as their type Lab-PC+ The signal across variable resistor 22 is an analog signal and hence the portion of the signal between leads 18 and wiper 24, which is applied to the A/D converter 26 and digital recorder 28, is also analog A computer 30 is connected to the output of the A/D converter 26, is preferably IBM compatible, and is programmed in a manner described hereinafter.

For example, preferably, the detector cell 10 is positioned adjacent an opposite wall of the sample container 8, and the emitter light source 4 positioned adjacent on opposite wall, so the light 6 emitted from the light source 4 passes through the container 8 The light source 4 is preferably selected to produce light 6 which can be absorbed by one or more components which are to be measured

The apparatus can be used to carry out coagulation studies in accordance with the invention. In accordance with a preferred embodiment of the present invention, the light source 4 may, for example, comprise a light emitting diode (LED) emitting a predetermined wavelength, such as for example, a wavelength of 660 nm, and the detector cell 10 may, for example, comprise a silicon photovoltaic cell detector Optionally, though not shown, a bar code reader may also be provided to read bar code labels placed on the sample container 8. The bar code reader may produce a signal which can be read by the computer 30 to associate a set of data with a particular sample container 8.

To carry out a coagulation study on blood plasma, the citrated blood is separated from the red blood cell component of the blood. Conventional methods of separation, which include centrifugation, may be employed. Also, the use of a container device such as that disclosed in our copending Application Serial No 09/724,529 filed on November 28, 2000 may also be used, and the method disclosed therein for reading the plasma volume relative to the sample volume may also be employed.

Illustrative of the apparatus and method according to the present invention is a coagulation study which can be carried out therewith. A reagent, such as Thromboplastin-Calcium (Tp-Ca) is added to the plasma sample which is maintained at about 37°C by any suitable temperature control device, such as a heated sleeve or compartment (not shown) The reagent addition is done by dispensing an appropriate amount of the reagent into the plasma portion of the blood.. The plasma portion may be obtained by any suitable separation technique, such as for example, centrifugation. Preferably, the container 8 is vented when reagent is added. The reagent for example, may comprise thromboplastin, which is added in an amount equal to twice the volume of the plasma- The reagent is mixed with the plasma. It is preferable to minimize air bubbles so as not to interfere with the results.. The plasma sample to which the reagent has been added is heated to maintain a 37°C temperature, which, for example, may be done by placing the container holding the plasma and reagent in a heating chamber (not shown).

Readings are taken of the optical activity of the components in the sample container 8.

Reaction kinematics may be studied by observing changes in the optical density of the plasma layer For example, an amount of reagent, such as Thromboplastin-Calcium (Tp-Ca), may be added to the plasma sample in the container. The plasma sample in the container may comprise a known amount of volume. Alternately, the plasma volume may be ascertained through the method and apparatus described in our copending Application Serial No 09/724,529 filed on November 28, 2000 A controlled amount of Tp-Ca reagent is added to the plasma sample. The amount of reagent added corresponds to the amount of plasma volume The detector cell 10 and emitter light source 4 are preferably positioned so the optical density of the plasma sample may be read, including when the reagent is added and the sample volume is thereby increased

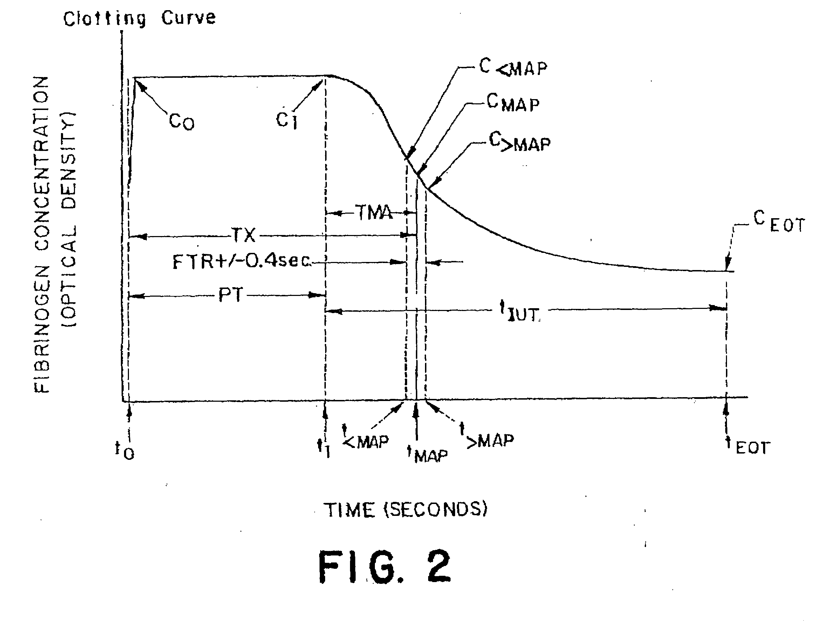

With the detection elements, such as the cell 10 and emitter 4, positioned to read the plasma sample and the reagents added thereto, the reaction analysis of the extended prothrombin time curve can be followed. Fig. 2 shows a graph of a plot of the various phases of the fibrinogen concentration occurring in a typical plasma clotting process. The change in optical density of the plasma level occurs after reagents have been added. The optical density of the plasma sample is monitored, as optically clear fibrinogen converts to turbid fibrin.

The coagulation study of thc type described above is used to ascertain the results shown in the graph plotted on Fig 2. The description of the analysis makes reference to terms, and symbols thereof, having a general description as used herein, all to be further described and all of which arc given in Table 1

TABLE 1

| SYMBOL | TERM | GENERAL DESCRIPTION |
|---|---|---|
| PT | Prothrombin Time | A period of time calculated from the addition of thromboplastin-calcium to a point where the conversion of fibrinogen to fibrin begins (i.e. the formation of the first clot). |
| TMA | Time to Maximum Acceleration | The time from PT to a point where the rate of conversion of fibrinogen to fibrin has reached maximum and begins to slow. |
| MAP | Maximum Acceleration Point | A point where the fibrinogen conversion achieves maximum acceleration and begins to decelerate. |
| EOT | End of Test | Point where there is no appreciable change in the polymerization of fibrin. |
| TX | Time to Map | Time to reach the Maximum Acceleration Point (MAP) from point of injection. |
| MNTX | Mean Normal Time to | The mean of the times of at least 20 normal |
| | Map | people to reach then Maximum Acceleration Point (MAP). |
| FTR | Fibrinogen Transformation Rate | The amount of fibrinogen converted during a time period from -1/2 TMA to +1/2 TMA. This is a percentage of the total Fibrinogen. |
| nFTR | new Fibrinogen Transformation Rate | The amount of fibrinogen converted during a time period from TMA -0.4 seconds to TMA +0.4 seconds. This is a percent of the total Fibrinogen. |
| ATF | Anticoagulation Therapy Factor | The calculated value used to monitor the uses of an anticoagulant without a need for an International Sensitivity Index (ISI) of a thromboplastin. |
| nATF | new Anticoagulation Therapy Factor | A replacement for the INR to provide a standardized system for monitoring oral anticoagulant therapy. |
| PR | Prothrombin Ratio | A value computed by dividing a sample PT by the geometric mean of at least 20 normal people (MNPT). |
| INR | International Normalized Ratio | A parameter which takes into account the various factors involved in anticoagulation therapy monitoring to provide a standardized system for monitoring oral anticoagulant therapy. |
| XR | Time to MAP Ratio | The value computed by dividing a sample "TX" by the geometric mean of at least 20 normal people "MNTX". |

Prior patents for obtaining an anticoagulant therapy factor (ATF) relied on the international normalized ratio (INR) system which was derived in order to improve the consistency of results from one laboratory to another The INR system utilized the calculation of INR from the equation:

$$INR = (PT_{patient}/PT_{geometric\ mean})^{ISI}$$

wherein the $PT_{patient}$ is the prothrombin lime (PT) as an absolute value in seconds for a patient, $PT_{geometric\ mean}$ is the mean, a presumed number of normal patients The international sensitivity index (ISI) is an equalizing number which a reagent manufacturer of thromboplastin specifies. The ISI is a value which is obtained through calibration against a World Health Organization primary reference thromboplastin standard- Local ISI (LSI) values have also been used to provide a further refinement of the manufacturer-assigned ISI of thc referenced thromboplastin in order to provide local calibration of the ISI value.

For illustration, the present invention can be employed for accurate determination of a new Anticoagulant Therapy Factor (nATF) from a human blood sample, for use during the monitoring of oral anticoagulant therapy, without the need for an ISI or LSI value, and without the need for an INR value As is known in the art, blood clotting Factors 1, II, V, VII, VIII, IX and X are associated with platelets (Bounamcaux. 1957); and, among these, Factors II, VII, IX and X are less firmly attached, since they are readily removed from the platelets by washing (Betterle, Fabris et al, 1977) The role of these platelet-involved clotting factors in blood coagulation is not, however, defined The present invention provides a method and apparatus for a new Anticoagulant Therapy Factor (nATF) which may be used for anticoagulant therapy monitoring without the need for INR.

The International Normalized Ratio (INR) is previously discussed in already incorporated reference technical articles entitled "PTs, PRs, ISIs and INRs: A Primer on Prothrombin Time Reporting Part I and II respectively," published November, 1993 and December, 1993 issues of Clinical Hemostasis Review. The illustrative example of an analysis which is carried out employing the present invention relics upon the maximum acceleration point (MAP) at which fibrinogen conversion achieves a maximum and from there decelerates, the time to reach the MAP (TX), and the mean normal time to MAP (MNTX), and a fibrinogen transformation rate (FTR), that is, the thrombin activity in which fibrinogen (FBG) is converted to fibrin to cause clotting in blood plasma.

More particularly, during the clotting steps used to determine the clotting process of a plasma specimen of a patient under observation, a thromboplastin (Tp) activates factor VII which, activates factor X, which, in turn, under catalytic action of factor V, activates factor II (sometimes referred to as prothrombin) to cause factor IIa (sometimes referred to as thrombin) that converts fibrinogen (FBG) to fibrin with resultant turbidity activity which is measured, in a manner as to be described hereinafter, when the reaction is undergoing simulated zero-order kinetics

From the above, it should be noted that the thromboplastin (Tp) does not take part in the reaction where factor IIa (thrombin) converts fibrinogen (FBG) to fibrin which is deterministic of the clotting of the plasma of the patient under consideration. The thromboplastin (Tp) only acts to activate factor VII to start the whole cascade rolling Note also that differing thromboplastins (Tps) have differing rates of effect on factor VII, so the rates of enzyme factor reactions up to II-IIa (the PT) will vary

Therefore, the prothrombin times (PTs) vary with the different thromboplastins (Tps) which may have been a factor that mislead authorities to the need of taking into account the International Normalized Ratio (INR) and the International Sensitivity Index (IST) to compensate for the use of different types of thromboplastins (Tps) during the monitoring of oral anticoagulant therapy. It is further noted, that thromboplastins (Tps) have nothing directly to do with factor IIa converting fibrinogen (FBG) to fibrin, so it does not matter which thromboplastin is used when the fibrinogen transformation is a primary factor

The thromboplastin (Tp) is needed therefore only to start the reactions that give factor IIa. Once the factor IIa is obtained, fibrinogen (FBG) to fibrin conversion goes on its own independent of the thromboplastin (Tp) used. Accordingly, for example in measuring the new anticoagulant therapy factor (nATF), one needs only take into account the determination of the new fibrinogen transformation rate (nFTR), the prothrombin time (PT) and the maximum acceleration point (MAP), all of which may be typically ascertained by the use of fibrinogen solutions,

The present method and apparatus has use, for example, in coagulation studies where fibrinogen (FBG) standard solutions and a control solution are employed, wherein the fibrinogen standard solutions act as dormant references to which solutions analyzed with the present invention are compared, whereas the control solution acts as a reagent that is used to control a reaction The fibrinogen standards include both high and low solutions, whereas the control solution is particularly used to control clotting times and fibrinogens of blood samples.

A fibrinogen (FBG) solution of about 10 g/l may be prepared from a cryoprecipitate The cryoprecipitate may be prepared by freezing plasma, letting the plasma thaw in a refrigerator and then, as known in the art, expressing off the plasma so as to leave behind the residue cryoprecipitate The gathered cryoprecipitate should contain a substantial amount of both desired fibrinogen (FBG) and factor VIII (antihemophilic globulin), along with other elements that arc not of particular concern to the present invention The 10 g/l fibrinogen (FBG) solution, after further treatment, serves as the source for the high fibrinogen (FBG) standard A 0 5 g/l fibrinogen (FBG) solution may then be prepared by a 1: 20 (10 g/l/20=0 5 g/l) dilution of some of the gathered cryoprecipitate to which may be added an Owren's Veronal Buffer (pH 735) - (known in the art) or normal saline solution and which, after further treatment, may serve as a source of the low fibrinogen (FBG) standard.

The fibrinogen standard can be created by adding fibrinogen to normal plasma in an empty container- Preferably, the fibrinogen standard is formed from a 1:1 fibrinogen to normal plasma solution. For example, 0 5 ml of fibrinogen and 0.5 ml of plasma can be added together in an empty container. Thromboplastin calcium is then added to the fibrinogen standard Preferably, twice the amount by volume of thromboplastin is added into the container per volume amount of fibrinogen standard which is present in the container The reaction is watched with the apparatus 10.

Then, 1 ml of each of the high (10 g/l) and low (0 5 g/l) sources of the fibrinogen standards may be added to 1 ml of normal human plasma (so the cryoprccipitate plasma solution can clot). Through analysis, high and low fibrinogen (FBG) standards are obtained. Preferably, a chemical method to determine fibrinogen (FBG) is used, such as, the Ware method to clot, collect and wash the fibrin clot and the Ratnoff method to dissolve the clot and measure the fibrinogen (FBG) by its tyrosine content- The Ware method is used to obtain the clot and generally involves collecting blood using citrate, oxalate or disodium ethylene diameantetracitate as anticoagulant, typically adding 10 ml to about 30 ml 0 85% or 0.90% sodium chloride (NaCl) in a flask containing 1ml M/5 phosphate buffer and 0.5 ml I % calcium chloride $CaCl_2$, and then adding 0.2 ml (100 units) of a thrombin solution. Preferably, the solution is mixed and allowed to stand at room temperature for fifteen minutes, the fibrin forming in less than one minute forming a solid gel if the fibrinogen concentration is normal. A glass rod may be introduced into the solution and the clot wound around the rod. See Richard J. Henry, M D , et al , Clinical Chemistry: Principals and Technics (2 nd Edition) 1974, Harper and Rowe, pp 458-459, the disclosure of which is incorporated herein by reference. Once the clot is obtained, preferably the Ratnoff method may be utilized to dissolve the clot and measure the fibrinogen (FBG) by its tyrosine content. See "A New Method for the Determination of Fibrinogen in Small Samples of Plasma", Oscar D Ratnoff, M D et al., J. Lab Clin Med, 1951: V 37 pp. 316-320, the complete disclosure of which is incorporated herein by reference- The Ratnoff method relies on the optical density of the developed color being proportional to the concentration of fibrinogen or tyrosine and sets forth a calibration curve for determining the relationship between optical density and concentration of fibrinogen. The addition of a fibrinogen standard preferably is added to the plasma sample based on the volume of the plasma.

As is known, the addition of the reagent Thromboplastin C serves as a coagulant to cause clotting to occur within a sample of citrated blood under test which may be contained in a container 8.. As clotting occurs, the A/D converter 26 of Fig- 1 will count and produce a digital value of voltage at a predetermined period, such as once every 0.05 or 0.01 seconds As more fully described in the previously incorporated by reference U-S- Patent No. 5,197,017 ('017), these voltage values are stored and then printed by the recorder as an array of numbers, the printing being from left to right and line by line, top to bottom. There are typically one hundred numbers in the five groups representing voltage values every second and hence, one line represents one-fifth of a second in time (20x0 .01 seconds). Individual numbers in the same column are twenty sequential numbers apart. Hence, the time difference between two adjacent numbers in a column is one-fifth of a second The significance of these recorded values may be more readily appreciated after a general review of the operating principles illustrated in Fig. 2 having a Y axis identified as Fibrinogen Concentration (Optical Density) and an X axis identified in time (seconds)

Fig. 2 illustrates the data point locations of a clotting curve related to a coagulation study which can be carried out with the present invention. In general, Fig. 2 illustrates a "clot slope" method that may be used in a blood coagulation study carried out in accordance with the present invention for determining a new anticoagulant therapy factor (nATF) The study which measures the concentration of the fibrinogen (FBG) in the plasma that contributes to the clotting of the plasma and uses the potentiophotometer apparatus of Fig 1 to provide an output voltage signal that is directly indicative of the fibrinogen (FBG) concentration in the plasma sample under test, is more fully discussed in the previously incorporated by reference U-S Patent 5,502,651 The quantities given along the Y-axis of Fig 2 are values (+and-) that may be displayed by the digital recorder 28. The "clot slope" method comprises detection of the rate or the slope of the curve associated with the formation of fibrin from fibrinogen. The "clot slope" method takes into account the time to maximum acceleration (TX) which is the point at which fibrinogen conversion achieves a maximum and from there decelerates

As seen in Fig. 2, at time $t_0$, corresponding to a concentration $c_0$, the thromboplastin/calcium ion reagent is introduced into the blood plasma which causes a disturbance to the composition of the plasma sample which, in turn, causes the optical density of the plasma sample to increase momentarily- After the injection of the reagent (the time of which is known, as to be described, by the computer 30), the digital quantity of the recorder 28 of Fig 1 rapidly increases and then levels off in a relatively smooth manner and then continues along until the quantity $c_1$ is reached at a time $t_1$. The time which elapses between the injection of thromboplastin at $t_0$ and the instant time $t_1$ of the quantity $c_1$ is the prothrombin time (PT) and is indicated m Fig 2 by the symbol PT

The optical density of the quantity $c_1$ directly corresponds to a specified minimum amount of fibrinogen (FBG) that must be present for a measuring system, such as the circuit arrangement of Fig. 1, to detect in the plasma sample that a clot is being formed. Further, all the quantities shown in Fig 2 are of optical densities that are directly correlatable to fibrinogen concentration values. The quantity $c_1$, may vary from one clot detection system to another, but for the potentiophotometer system of Fig I, this minimum is defined by units of mass having a value of about 0 05 grams/liter (g/l).

The detection of this first predetermined quantity $c_1$ is shown in Fig 2 to occur at an instant time $t_1$ which is the start of the clotting process being monitored with the apparatus of Fig. 1 for determining the new anticoagulant therapy factor (nATF) The time $t_1$ is the beginning point of the fibrinogen formation, that is, it is the point that corresponds to the beginning of the acceleration of the fibrinogen conversion that lasts for a predetermined time, preferably about 1.5 seconds. This $t_1$ point is determined by a real time analysis of the optical density data accumulated during testing The time duration of at least 15 seconds allows a sufficient amount of delay time to eliminate any false responses due to noises created by initial mixing of the reagent into the sample or bubbles within the sample under test. This 1 5 second duration helps determine the beginning point ($t_1$) of the fibrinogen conversion in spite of any bubbles or artifacts that might be present for short durations. These noise producers might otherwise be erroneously interpreted as early clots and might lead to a correspondingly false response by the instrument performing the measuring Accordingly, the noise time may be greater or less than the 1. 5 seconds, as needed, and can be adjusted by programming the computer 30.

The acceleration of the fibrinogen conversion that occurs within the about 1.5 second duration, is shown in Fig. 2. The acceleration of the fibrinogen conversion proceeds from time $(t_1)$ and continues until a time $t_{MAP}$, having a corresponding quantity $c_{MAP}$. The time $t_{MAP}$, as well as the quantity $C_{MAP}$, is of primary importance because it is the point of maximum acceleration of the fibrinogen (FBG) to fibrin conversion and is also the point where deceleration of fibrinogen (FBG) to fibrin conversion begins. Further, the elapsed time from $t_0$ to $t_{MAP}$ is a time to maximum acceleration from reagent injection (TX), shown in Fig 2 Preferably, the conversion of fibrinogen to fibrin is quantified every 0 1 seconds The time to maximum acceleration from reagent injection (TX) is defined as the point on the clotting curve time line where this conversion has reached its maximum value for the last time, simulating a zero-order kinetic rate. To facilitate ascertainment of the location point of the last maximum value, the delta value of two points at a fixed interval, such as for example 2 5 seconds, is measured until this value begins to decrease This value is tracked for a period of time, such as for example five seconds, after the first decreasing value has been determined. This facilitates ascertainment of the last point of what may be referred to as a simulated zero-order kinetic rate

The quantity $C_{MAP}$ and the time $t_{MAP}$ define a maximum acceleration point (MAP) and are shown in Fig. 2 as having predetermined ranges starting prior to maximum acceleration point (MAP) and ending after the maximum acceleration point (MAP), with the difference covered by the overall range defining the new fibrinogen transformation rate (nFTR), which is also shown in Fig. 2 and has a band of +/- 0 4 seconds Fibrin formation, after a short lag phase before the MAP, occurs for a period of time, in a linear manner. Fibrinogen (FBG) is in excess during this lag phase, and fibrin formation appears linear up to the MAP. Referring to Fig 2, the new fibrinogen transformation rate (nFTR) is defined as the fibrinogen converted during the period from 0.4 seconds before the maximum acceleration point (MAP) to 0-4 seconds after the maximum acceleration rate (MAP) divided by the total fibrinogen. This differential provides the percent of the total fibrinogen converted. The new fibrinogen transfer rate is expressed by the following formula:

$$nFTR=IUX/IUT$$

where IUX is the change in optical density at the time $t_{MAP}$ - 0.4 seconds to the optical density at time $t_{MAP}$ + 0.4 seconds; and wherein IUT = the change in optical density at the time $t_1$ to the optical density measured at time $t_{EOT}$, where time $t_{EOT}$ is the end of the test (EOT). The (IUX) represents the fibrinogen (FBG) for MAP -0.4 seconds to MAP +0 4 seconds (that is the fibrinogen (FBG) converted from $t<_{MAp}$ to $t>_{MAP}$ on Fig. 2) The (IUT) represents fibrinogen converted from $C_1$ to $C_{EOT}$ (that is the fibrinogen converted from $t_1$ to $t_{EOT}$, see Fig. 2).

The maximum acceleration ratio (XR) is calculated to arrive at the new anticoagulation therapy factor (nATF). The maximum acceleration ratio (XR) is defined as the time to maximum acceleration from reagent injection (TX) divided by the mean normal TX value of a number of presumed normal specimens (MNTX) For example, the mean normal TX value may be derived based on the value of 20 or more presumed normal specimens The maximum acceleration ratio (XR) may be expressed through the following formula

$$XR = TX/MNTX$$

The clotting curve of Fig 2 illustrates the values ascertained in arriving at the new anticoagulation therapy factor (nATF) The new anticoagulation therapy factor (nATF) is preferably expressed by the following formula:

$$nATF = XR^{(2-nFTR)}$$

In the above equation, in the exponent, the value 2 is the logarithm of the total fibrinogen, which, as expressed in terms of the optical density, is 100% transmittance, the log of 100 being 2. The new fibrinogen transformation rate (nFTR) is a percentage of the total fibrinogen converted to fibrin during a fixed period, such as for example, from 0 4 seconds before the (MAP) to 0.4 after the (MAP), divided by the total fibrinogen Establishing the exponent in the equation $nATF = XR^{(2-nFTR)}$ is obtained by subtracting the (nFTR), which is a linear value, from 2, which is the linear equivalent of an optical density (O.D.) of 100% (the log of 100), as the output of the analytical spectrophotometer instrument (POTENS+) is linear. While generally, a spectrophotometer has an output, that is optical density (O D ) which is logarithmic, the relationship of the optical density (O.D.) to (2-log %T) is direct (i.e. linear), where %T is the percent transmittance. The exponent, (2 - nFTR), and the direct expression, (2 - log %T), arc comparable. The linear machine output of the analytical instrument (such as for example the POTENS+) is compensated for by the logarithmic analytical instrument (i e- spectrophotometcr) output. That is, a linear-log relationship of the analytical instruments is converted to the log-linear of the instruments' chemistries wherein (2 - nFTR) and (2 - log % T) are compared. The exponent (2 - nFTR) is analogous to (2 - log %T), which equals the optical density (O.D ) in photometry The number 2 (the log of 100) represents all of the light incident on the material being measured, and "log %T" represents the number for the amount of light transmitted For the exponent expression (2 - nFTR) the 2 represents the total amount of fibrinogen (FBG) involved, and, the new fibrinogen transfer rate (nFTR) is the amount of fibrinogen (FBG) measured at a predetermined time period from the inflection point of the clotting curve, such as for example a time period of ±0.4 seconds, divided by the total fibrinogen (FBG) For photometry, (2 - log %T) is 2 minus a number and for the new anticoagulant therapy factor (nATF), it is also 2 minus a number. The numbers involved in the ultimate expression to obtain the new anticoagulant therapy factor (nATF) and to obtain the optical density (O.D ) are not logarithms, but rather, are natural numbers.

The new anticoagulation therapy factor (nATF) does not require an ISI value, as was previously used to determine anticoagulation therapy factors. The new anticoagulation therapy factor (nATF) uses for its ascertainment the values extracted from the clotting curve (see Fig 2). The new fibrinogen transformation rate (nFTR) is of primary importance in the coagulation study exemplified herein because it is one of the three parameters that determine the new anticoagulant therapy factor (nATF) of the present invention with the other two being the time to maximum acceleration from reagent injection (TX), and the maximum acceleration point (MAP). The predetermined range may be from about 0 1 seconds to about 5 0 seconds on each side of the maximum acceleration point (MAP) shown in Fig. 2 so that the new fibrinogen transformation rate (nFTR) may cover an overall difference from about 0 2 seconds to about 10.0 seconds. It is particularly preferred that the predetermined range is from about 0.4 seconds before the maximum acceleration point (MAP) to about 0 4 seconds after the maximum acceleration point (MAP)

The deceleration of fibrinogen (FBG) to fibrin conversion continues until a quantity $C_{EOT}$ is reached at a time $t_{EOT}$ The time $t_{EOT}$ is the point where the deceleration of the fibrinogen (FBG) to fibrin conversion corresponds to a value which is less than the required amount of fibrinogen (FBG) that was present in order to start the fibrinogen (FBG) to fibrin conversion process Thus, because the desired fibrinogen (FBG) to fibrin conversion is no longer in existence, the time $t_{EOT}$ represents the ending point of the fibrinogen (FBG) to fibrin conversion in accordance with the coagulation study exemplified herein, which may be referred to as the end of the test (EOT) The fibrinogen (FBG) to fibrin conversion has a starting point of $t_1$ and an ending point of $t_{EOT}$ The differential of these times, $t_1$ and $t_{EOT}$, define a second delta (IUT)

The significance of the points $t_1$, and $t_{EOT}$ are not the times at which they occur, but rather the difference in the optical density of the quantities $c_1$ and $C_{EOT}$ occurring at the respective times $t_1$ and $T_{EOT}$. This difference is defined herein as the delta optical density of the "clot slope" method and is of importance to determining the new anticoagulant therapy factor (nATF) The "clot slope" method that gathers typical data as shown in Fig. 2 has four critical parameters-The first is that the initial delta optical density of substance being analyzed should be greater than about 0-05 g/l in order for the circuit arrangement of Fig 1 to operate effectively. Second, the acceleration fibrinogen (FBG) to fibrin conversion should be increasing for a minimum period of about 1..5 seconds so as to overcome any false reactions created by bubbles. Third, the total delta optical density (defined by the difference in quantities $c_1$ and $C_{EOT}$) should be at least three (3) times the instrument value in order to perform a valid test, i e , (3)*(0 05 g/l)=0 15 g/l. Fourth, the fibrinogen (FBG) to fibrin conversion is defined, in part, by the point ($t_{EOT}$) where the deceleration of conversion becomes less than the instrument value of about 0.05 g/l that is used to detect the clot point ($t_1$). As with most clot detection systems, a specific amount of fibrinogen needs to be present in order to detect a clot forming. Adhering to the four given critical parameters is an example of how the present apparatus and method may be used to carry out a coagulation study to determine a specific quantity of fibrinogen. In order for that specific amount of fibrinogen to be determined, it is first necessary to detect a clot point ($t_1$). After that clot point ($t_1$) is detected, it logically follows that when the fibrinogen conversion becomes less than the specific amount (about 005 g/l for the circuit arrangement of Fig. 1), the end point ($t_{EOT}$) of the fibrinogen conversion has been reached.

The gathering, storing, and manipulation of the data generally illustrated in Fig 2, is primarily accomplished by computer 30 of Fig 1 that receives digital voltage values converted, by the A/D converter 26, from analog voltage quantities of the photocell 10 detection means.

The preferred IBM-compatible computer 30 of Fig 1 stores and manipulates these digital values corresponding to related data of Fig 2 and is preferably programmed as follows :

(a) with citrated blood, such as described above, in a container, the computer 30, as well as the recorder 28, sequentially records voltage values for a few seconds before injection of thromboplastin. As previously discussed, thromboplastin (tissue factor) is one of the factors in the human body that causes blood to clot Prothrombin is another. Fibrinogen is yet another. Before injection of the thromboplastin, the output from the A/D converter 26 is relatively constant. When thromboplastin is injected into the plasma sample in the container, a significant and abrupt change occurs in the recorded voltage values of both the computer 30 and the recorder 28. This abrupt change is recognized by both the recorder 28 and, more importantly, by the computer 30 which uses such recog-

nition to establish $t_0$ already discussed with reference to Fig. 2. The computer 30 may be programmed so as to correlate the digital quantities ofthe A/D converter 26 to the analog output of the detector means photocell 10 which, in turn, is directly correlatable to the fibrinogen (FBG) concentration g/l of the sample of blood discussed with reference to Fig 2;

(b) the computer 30 may be programmed to look for a digital quantity representative of the previously discussed critical quantity $C_1$, and when such occurs, record its instant time $t_1$ (The time span between $t_0$ and $t_1$ is the prothrombin time (PT), and has a normal duration of about 12 seconds, but may be greater than 30 seconds);

(c) following the detection of the critical quantity $C_1$, the computer 30 may be programmed to detect for the acceleration of fibrinogen (FBG) to fibrin conversion. The computer 30 is programmed to detect the maximum acceleration quantity $C_{MAP}$ and its time of occurrence $t_{MAP}$ Furthermore, the computer detects the quantity $C_{EOT}$ occurring at time $t_{EOT}$ Typically, it is important that the rate of fibrin formation increase for at least 1 5 second following the occurrence of ($t_1$);

(d) following the detection of the maximum acceleration quantity $C_{MAP}$ and the time $t_{MAP}$ both of which define the maximum acceleration point (MAP), the computer 30 is programmed to determine the new fibrinogen transformation rate (nFTR) covering a predetermined range starting prior to the maximum acceleration point (MAP) and ending after the maximum acceleration point (MAP) The elapsed time from $t_0$ to $t_{MAP}$ is the time to maximum acceleration (TMA) shown in Fig 2

The new fibrinogen transformation rate (nFTR) has an upwardly rising (increasing quantities) slope prior to the maximum acceleration point (MAP) and, conversely, has a downwardly falling (decreasing quantities) slope after the maximum acceleration point (MAP). The computer 30 is programmed to allow for a predetermined range defining the new fibrinogen transformation rate (nFTR) which preferably may be from about 0.4 seconds on each side of the maximum acceleration point (MAP) so that the new fibrinogen transformation rate (nFTR) may cover an overall difference from about 0.8 seconds;

(e) following the detection of the acceleration of fibrinogen conversion, the computer 30 is programmed to detect for a deceleration of the fibrinogen conversion, wherein the fibrinogen concentration decreases from its third predetermined quantity $C_{MAP}$ to a fourth predetermined quantity $C_{EOT}$ having a value which is about equal but less than the first quantity $c_j$. The difference between the first quantity $c_1$ and the quantity $C_{EOT}$, defines a delta (TUT);

(f) the computer 30 manipulates the collected data of (a); (b); (c); (d) and (e) above, to determine the new fibrinogen transfer rate (nFTR) based on the principle that if a required amount (e.g., 0.05 g/l) of fibrinogen concentration $c_1$ is first necessary to detect a clot point ($t_1$); then when the fibrinogen concentration ($c_{EOT}$) becomes less than the required amount $c_1$, which occurs at time ($t_{EOT}$), the fibrinogen end point has been reached- More particularly, the required fibrinogen concentration $c_1$ is the starting point of fibrinogen conversion of the clotting process and the less than required fibrinogen concentration $C_{EOT}$ is the end point of the fibrinogen conversion of the clotting process Thus, the duration of the fibrinogen conversion of the clotting process of the present invention is defined by the time period between $t_1$ and $t_{EOT}$ and is generally indicated in Fig 2 as $t_{IUT}$, and the difference between the corresponding concentrations $c_1$ and $C_{EOT}$ is used to define a delta IUT The computer now has the information needed to determine the new fibrinogen transfer rate (nFTR) which is expressed by the following formula:

$$nFTR = IUX/IUT \tag{1}$$

where IUX is the change in optical density at the time $t_{MAP}$ - 0-4 seconds to the optical density at time $t_{MAP}$ + 0.4 seconds; and

wherein IUT is the change in optical density at time $t_1$ to the optical density measured at time $t_{EOT}$, where time $t_{EOT}$ is the end of the test (EOT).

(g) data collected is manipulated by the computer 30 to calculate the maximum acceleration ratio (XR), which is expressed as TX divided by the mean normal TX value of at least 20 presumed normal specimens (MNTX):

$$XR = TX/MNTX \tag{2}$$

The MNTX value may be ascertained and stored in the computer for reference.

(h) the computer 30 now has the information needed to determine the nATF, which typically is expressed as:

$$nATF = XR^{(2-nFTR)} \qquad (3)$$

where, in the exponent, the value 2 is the logarithm of the total fibrinogen, which, as expressed in terms of the optical density, is 100% transmittance, the log of 100 being 2

The new anticoagulation therapy factor (nATF) does not require an ISI value, as was previously used to determine anticoagulation therapy factors. The new anticoagulation therapy factor (nATF) uses for its ascertainment the values extracted from the clotting curve (see Fig 2), in particular (nFTR), and (TX). In carrying out coagulation studies, the new anticoagulant therapy factor (nATF) may replace INR in anticoagulant therapy management- More particularly, the computer 30 has knowledge of the new fibrinogen transformation rate (nFTR)

It should now be appreciated that the present invention provides an apparatus and method for obtaining a new anticoagulant therapy factor (nATF) without encountering the complications involved with obtaining the prior art quantities International Normalized Ratio (INR) and International Sensitivity Index (ISI)

The new anticoagulant therapy factor (nATF) preferably is a replacement for the International Normalized Ratio Existing medical literature, instrumentation, and methodologies are closely linked to the International Normalized Ratio (INR) The nATF was compared for correlation with the INR by comparative testing, to INR quantities, even with the understanding that the INR determination may have an error of about thirteen (13) % which needs to be taken into account to explain certain inconsistencies

The hereinbefore description of the new anticoagulant therapy factor (nATF) does correlate at least as well as, and preferably better than, studies carried out using the the International Normalized Ratio (INR).

The computer 30 may be used to manipulate and derive the quantities of expression (3) utilizing known programming routines and techniques The data collected by a computer 30 may bc used to manipulate and derive the new anticoagulant therapy factor (nATF) of expression (3) Similarly, one skilled in the art, using known mathematical techniques may derive the nFTR and the mean normal TX value (MNTX) of expression (2) which, in turn, are used to determine the new anticoagulant therapy (nATF) of expression (3) The accuracy of these quantities is dependent, in part, on the number of specimens used, that is, the number of stable patients; wherein for the practice of this embodiment of the present invention, with reference to a calibration procedure, a number of at least twenty (20) of stable patients is preferably used and which is in agreement with that used in the art to establish a population sampling standard, such as disclosed in the previously incorporated by reference technical article of L Poller ct al In order to compare the results obtained with the nATF to those obtained using the INR, the following data analysis was undertaken, using the following instrument (i) Organon-Teknika MDA®180 Coagulation Analyzer using Simplastin L thromboplastin. This was used to determine the INR's for the first part of the analysis; and the (ii) POTENS+ instrument using Thromboplastin-C Plus (TPC) to determine nATF's to be compared with the MDA INR's. Ng comparisons were made between the INR's and nATF's obtained with the respective Instruments See Article *"Highly Sensitive Thromboplastins Do Not Improve INR Precision* ", Ng et al , Vol. 109, No 3, pp. 338-346, AM. J. Clinical Pathology, March 1998.

The POTENS+ was used to determine, simultaneously, TNR's and nATF's on the injection of each thromboplastin. Four different thromboplastins were used, providing four pairs of INR-NATF data. Ng comparisons were made on each pair of data. In conducting the analysis two POTENS+ instruments (GVCH and MGPL) were used Since both instruments, MDA-180 and POTENS+, are totally different optical coagulation instruments, it was anticipated that the Ng mismatches and the correlation coefficients and slopes would be worse with MDA-180 Simplastin L vs POTENS+-TPC instruments than with the same results all derived on POTENS+ only, even though with the four different thromboplastins In the latter case, it is presumed that the only variable is the four different thromboplastins

The following thramboplastins were used:

Table 2.

| THROMBOPLASTINS | | | | |
|---|---|---|---|---|
| | Thromboplastin | Lot No. | ISI | MNPT |
| TPC | Dade® Thromboplastin C Plus Dade Behring Marburg, Germany | Lot 527053 | 2.12 | 11.9 |
| INN | Dade® Innovin™ Baxter Diagnostics Inc Deerfield, IL | Lot/Ch-B.-TFS-231 | 1.02 | 9.5 |
| SIG | Sigma Diagnostics® St. Louis, MO | Lot 124H170 | 2.51 | 10.8 |
| PHT | Thromboplastin-D Pacific Haemostasis Huntersville, NC | Lot 357X06 | 1.99 | 12.4 |

Table 2. (continued)

| THROMBOPLASTINS | | | | |
|---|---|---|---|---|
| | Thromboplastin | Lot No. | ISI | MNPT |
| MDA | MDA® Simplastin® I. Organon-Teknika Durham, NC | Lot/Ch-B 50052 | 2.00 | 12.0 |

Table 3.

| COMPARATIVE RESULTS | | | | | | |
|---|---|---|---|---|---|---|
| Comparison | n | r | m | b | Ng | Poller |
| GVCH nATF vs. MDA INR (all data) | 336 | 0.918 | 0.882 | 0.302 | 58/336= 17.3% mismatches | 30/336=8.9% |
| GVCH nATF vs. rNR (excluding all TNRs >4 5) | 312 | 0.951 | 1.007 | 0.41 | 49/312= 15.7% mismatches | 17/312=5.4% |
| MGPL nATF vs. MDA INR (all data) | 336 | 0.933 | 0.928 | 0.273 | 63/336= 18.8% mismatches | 42/336=125% |
| MGPL nATF vs. MDA INR (excluding all INRs >4.5 | 312 | 0.952 | 1.089 | -0.061 | 51/312= 16.3% mismatches | 32/312=10.3% |
| GVCH nΛTF vs. GVCH INR (all data) | 336 | 0.992 | 1.012 | 0.002 | 24/336= 7.1% mismatches | 2/336=0.6% |
| GVCH nATF vs. GVCH INR (excluding all INR5 >4 5) | 312 | 0.986 | 0.990 | 0.042 | 21/312 6.7% mismatches | 2/312=0.2% |
| MGPL nATF vs. MGPL INR (call data) | 336 | 0.990 | 1.001 | 0.001 | 23/336= 6.8% mismatches | 0/336=0% |
| MGPL nATF vs. INR (excluding all INRs >4 5) | 312 | 0.984 | 1.005 | 0.001 | 21/312= 6.7% mismatches | 0/312-0% |

The instrumentation manual (Organon-Teknika Operator Manual) for the MDA-180® Coagulation Analyser states that INR values above a level of 4 5 are of limited utility, so calculations in Table 3, above, were done both, inclusive and exclusive, of the >4.5 MDA INR's

In *"Highly sensitive Thromboplastins Do Not Improve INR Precision*, Ng et al ,Vol 109, No 3, pp 33 8-346, Am J Clinical Pathology, March 1998, there is reported an average of 24% mismatches in Table 4 of the article with a total of 414 pairs on four different thromboplastins The present results achieved with the method and apparatus according to the present invention are much more favorable, and the comparison analysis carried out for the present invention tests the POTENS+ nATFs more severely than where both INR and nATF are done in one instrument, POTENS+, simultaneously as is demonstrated as follows:

POTENS+ GVCH INR vs GVCH nATF Ng results: a) 24 mismatches/336 pairs = 7.1% mismatches, with Poller data yielding 2 mismatches/336 pairs = 0.6%. For data where INR values < 4.6, there were 21 mismatches/312 pairs = 6.7%, with Poller data yielding 2 mismatches/312 pairs = 0 2%. b) POTENS+ MGPL INR vs MGPL nATF Ng results: 23 mismatches/336 pairs = 6.8% mismatches, with Poller data yielding 0 mismatches/336 pairs = 0% For data where INR's < 4 6, there were 21 mismatches/312 pairs = 6.7%, with Poller data yielding 0 mismatches/312 pairs = 0%.

INR-nATF pairs all run on POTENS+ GVCH and POTENS+ MGPL. (The Ng data and INR-nATF pairs for direct individual comparisons have been presented above in Table 3.) (All of these nATF values have been corrected similarly to the way the CATF was made the MATF in *Warfarin Monitoring By An Anticoagulant Therapy Factor (ATF),* W.E. Carroll, R.D. Jackson and T.A. Carroll, in Res Commun. Molec Pathol. Pharmacol-, Vol. 101, No. 2, August 1998) Now, *mean y* is subtracted from the values to be corrected, then this number is divided by the slope of x,y and finally, *mean x* is added back to give a corrected nATF

A comparison was also made for the data run on the POTENS+ instrument in 1997 that formed the basis for the results set forth in U S Patent 5,981,285 and the study reported in *Warfarin Monitoring By An Anticoagulant Therapy Factor (ATF),* W E. Carroll, R.D Jackson and T A Carroll, in Res. Commun Molec Pathol Pharmacol , Vol 101, No.2, August 1998.

Table 4A

| SINGLE INSTRUMENT COMPARISON OF DATA | | | | | | | | |
|---|---|---|---|---|---|---|---|---|
| | n | r | m | *mean x* | mean *y* | Ng | ISI | MNPT |
| a) TPC | 92 | 0.97 | 0.85 | 2.1 | 2.1 | 7/92= 7.6% mismatches | 2.12 | 11.9 |
| b) INN | 92 | 0.97 | 5.5 | 2.6 | 7.4 | 70/92 = 76.1% mismatches | 1. 02 | 9.5 |
| c) SIG | 92 | 0.96 | 0.88 | 2.4 | 2.4 | 13/92 = 14.1% mismatches | 2.51 | 10.8 |
| d) PHT | 92 | 0.99 | 0.91 | 2.3 | 2.3 | 2/92 = 2.2% mismatches | 1.99 | 12.4 |

Table 4B

| SINGLE INSTRUMENT COMPARISON OF DATA | | | | | | | | |
|---|---|---|---|---|---|---|---|---|
| | n | r | m | *mean* x | *mean* y | Ng. | | ISI MNPT (sec.) |
| a.) TPC corrected | 92 | 0.97 | 1.00 | 2.1 | 2.1 | 8/92= 8.7% mismatches | 2.12 | 11.9 |
| b.) INN corrected | 92 | 0.97 | 1.00 | 2.6 | 2.6 | 10/92= 10.9% mismatches | 1.02 | 9.5 |
| c.) SIG corrected | 92 | 0.96 | 1.00 | 2.4 | 2.4 | 16/92= 17.4% mismatches | 2.51 | 10.8 |
| d.) PHT corrected | 92 | 0.99 | 1.00 | 2.3 | 2.3 | 3./92= 3.3% mismatches | 1.99 | 12.4 |

It is to be noted that, with the same instrument, results are better than when different (MDA and POTENS+) instruments are used Of the different thromboplastins, Innovin (LNN) is prepared by recombinant technology from human material in Escherechia coli bacteria. The other three thromboplastins are made from rabbit brain. As listed in Table 4A, above, the ISI's vary from 1.02 to 2.51 It is anticipated that a single instrument's results would compare more favorably than the results from multiple instruments. Table 4B, above, lists single instrument comparison for corrected data

While the invention has been described with reference to specific embodiments, the description is illustrative and is not to be construed as limiting the scope of the invention For example, although described in connection with body fluids of a human, the present invention has applicability to veterinary procedures, as well, where fluids are to be measured or analyzed Various modifications and changes may occur to those skilled in the art without departing from the spirit and scope of the invention described

**Claims**

1.  A method of determining a new anticoagulant therapy factor (nATF) comprising the steps of developing a series of analog electrical voltage signals having voltage amplitudes proportional to an optical density of a liquid sample containing fibrinogen;

    a. converting the developed analog voltage signals into a series of digital voltage value signals;
    b. adding a coagulant into the liquid sample, thereby producing an abrupt change in the optical density of the liquid sample, said abrupt change producing an abrupt change in the amplitude of the electrical analog signals which, in turn, produces an abrupt change in the value of said digital voltage signals, the value of said digital voltage signals being directly indicative of fibrinogen concentration in the liquid sample;
    c. recording an instant time $t_o$ of said abrupt change in said value of said digital voltage signal;
    d monitoring said voltage digital signal values for a first predetermined fibrinogen concentration quantity $c_1$;
    e. recording an instant time $t_1$ and the value of the voltage digital signal of said first predetermined fibrinogen concentration quantity $c_1$;
    f monitoring said voltage digital signal values for further fibrinogen concentration quantities;
    g. recording an instant time $t_{MAP}$ and the value of the voltage digital signal of said predetermined fibrinogen concentration quantity $C_{MAP}$;

h. recording an elapsed time between to and $t_{MAP}$ which defines a time to maximum acceleration from coagulant injection in step (c);

i monitoring for a differential change in the voltage digital signal values that include a predetermined fibrinogen concentration quantity $C_{MAP}$;

j. said fibrinogen concentration quantity $C_{MAP}$ and said time $t_{MAP}$ defining a maximum acceleration point (MAP) and a time to maximum acceleration from coagulant injection (TX) being measured as the elapsed time from the time of the coagulant injection to to the time to maximum acceleration $t_{,MAP}$, and each of the quantity $C_{MAP}$ and said time $T_{MAP}$ having a predetermined range starting prior to, at a time $t_{<MAP}$, and ending after said maximum acceleration point (MAP), at a time $t_{>MAP}$;

k. monitoring voltage digital signal values at times $t_{<MAP}$ and $t_{>MAP}$ for respective predetermined fibrinogen concentration quantities $C_{<MAP}$ and $C_{>MAP}$ with the difference between quantities $C_{<MAP}$ and $C_{>MAP}$ being a first differential IUX,

l. monitoring voltage digital signal values at time $t_{EOT}$ and recording an instant time $t_{EOT}$ the value of the voltage digital signal of said predetermined fibrinogen concentration quantity $t_{EOT}$, with the difference between quantities $c_1$ and $C_{EOT}$ being a second differential IUT, the first differential being divided by the second differential to define a percentage of the total voltage digital signal value change covered by an overall range defining a new fibrinogen transformation rate (nFTR), where nFTR = IUX/IUT,

wherein a maximum acceleration ratio (XR) is determined by the time to maximum acceleration from the coagulant injection (TX) divided by a mean normal TX value (MNTX) of a sample of presumed normal patients;

wherein the new anticoagulant therapy factor (nATF) is expressed by the following relationship:

$$nATF = XR^{(2-nFTR)}$$

2. Method according to claim 1, wherein TX represents a time interval of the mean of a sample of presumed normal patients.

3. Method according to any one of the preceding claims, wherein the sample of mean normal patients is about 20 patients.

4. Method according to any one of the preceding claims, wherein the MNTX is the mean of the TX of the plurality of samples from at least twenty (20) normal people.

5. Method according to any one of the preceding claims, wherein the sample of mean normal patients is about equal to or greater than 20 patients.

6. Method according to any one of the preceding claims, wherein the predetermined range starting prior to and ending after said maximum acceleration point (MAP) is from about a time $t_{<MAP}$ occurring 0,4 seconds prior to time $t_{MAP}$ to a time $t_{>MAP}$ occurring 0,4 seconds after the time $t_{MAP}$.

7. Method according to any one of the preceding claims, wherein said liquid sample is blond plasma.

8. Method according to any one of the preceding claims, wherein the coagulant which is injected into the sample is thromboplastin with calcium ion.

9. Method according to any one of the preceding claims, wherein the analog electrical voltage signal is developed by transmitting a light beam through a plasma sample and sensing the variations in light passing therethrough to develop corresponding variations in the electrical signal produced.

10. An apparatus for determining a new anticoagulant therapy factor (nATF) comprising:

a. means including a light source, a test tube, a photocell, a battery, and a variable resistor all for developing an analog electric voltage signal having an amplitude proportional to an optical density of a liquid sample containing fibrinogen;

b. means including an A/D converter and a computer both cooperating for converting and recording the developed analog signal into a series of digital voltage signal values;

c. means for injecting a coagulant into a liquid sample, thereby producing an abrupt change in the optical density of the liquid sample, said abrupt change producing a change in the amplitude of the electrical analog signals, which, in turn, produces an abrupt change in the value of said digital voltage signals, the value of said digital voltage signals being directly indicative of fibrinogen concentration in the liquid sample;

d. means for recording an instant time t, of said abrupt change in said value of said digital voltage signal;

e. means, including a computer, for monitoring said voltage digital signal values for a first predetermined fibrinogen concentration quantity $c_1$;

f. means for recording an instant time t, and the value of the voltage digital signal of said first predetermined fibrinogen concentration quantity $C_1$;

g. means, including a computer, for monitoring said voltage digital signal values for further fibrinogen concentration quantities;

h. means for recording an instant time $t_{MAP}$ and the value of the voltage digital signal of said predetermined fibrinogen concentration quantity $C_{MAP}$;

i. means for recording an elapsed time between $t_0$ and $t_{MAp}$ which defines a time to maximum acceleration from coagulant injection in step (c);

j means, including said computer, for monitoring for a differential change in the voltage digital signal values that include a predetermined fibrinogen concentration quantity $C_{MAP}$;

k. said fibrinogen concentration quantity $C_{MAP}$ and said time $t_{MAP}$ defining a maximum acceleration point (MAP) and a time to maximum acceleration from coagulant injection (TX) being measured as the elapsed time from the time of the coagulant injection $t_0$ to the time to maximum acceleration $t_{MAP}$, and each of the quantity $C_{MAP}$ and said time $t_{MAP}$ having a predetermined range starting prior to, at a time $t_{<MAP}$, and ending after said maximum acceleration point (MAP), at a time $t_{>MAP}$;

l. means, including said computer, for monitoring voltage digital signal values at times $t_{<MAP}$ and $t_{>MAP}$ for respective predetermined fibrinogen concentration quantities $C_{<MAP}$ and $C_{>MAP}$, and for calculating the difference between quantities $C_{<MAP}$ and $C_{>MAP}$ to provide a first differential (IUX);

m. means, including said computer, for monitoring voltage digital signal values at time $t_{EOT}$ and recording an instant time $t_{EOT}$ the value of the voltage digital signal of said predetermined fibrinogen concentration quantity $C_{EOT}$, and for calculating the difference between quantities $c_1$ and $C_{EOT}$ to provide a second differential (IUT);

n. means, including said computer, for dividing the first differential (IUX) by the second differential (IUT) to define a percentage of the total voltage digital signal value change covered by an overall range defining a new fibrinogen transformation rate (nFTR), where nFTR = IUX/IUT;

o means, including said computer, for dividing the time to maximum acceleration from the coagulant injection (TX) by a mean normal TX value of a sample of presumed normal patients to provide a maximum acceleration ratio (XR) which is factored to the (2-nFTR) power with the product thereof being the new anticoagulant therapy factor (nATF) is expressed by the following relationship:

$$nATF = XR^{(2-nFTR)}$$

**11.** The apparatus according to claim 10, wherein said liquid sample is blood plasma.

**12.** The apparatus according to any one of the claims 10 to 11, wherein said coagulant which is injected into the sample is thromboplastin with calcium ion.

**13.** The apparatus according to any one of the claims 10 to 12, wherein the analog electrical voltage signal is developed by transmitting a light beam through a plasma sample and sensing the variations in light passing therethrough to develop corresponding variations in the electrical signal produced.

FIG. 1

EP 1 473 568 A2

FIG. 2